(19) Europäisches Patentamt
European Patent Office
Office européen des brevets

(11) Veröffentlichungsnummer: **0 162 247**
**B1**

(12) # EUROPÄISCHE PATENTSCHRIFT

(45) Veröffentlichungstag der Patentschrift: **26.08.87**

(51) Int. Cl.⁴: **C 07 D 231/12**

(21) Anmeldenummer: **85103982.6**

(22) Anmeldetag: **02.04.85**

(54) **Verfahren zur Herstellung von Pyrazolen.**

(30) Priorität: **25.04.84 DE 3415385**

(43) Veröffentlichungstag der Anmeldung: **27.11.85 Patentblatt 85/48**

(45) Bekanntmachung des Hinweises auf die Patenterteilung: **26.08.87 Patentblatt 87/35**

(84) Benannte Vertragsstaaten: **BE CH DE FR GB IT LI**

(56) Entgegenhaltungen:
**EP - A - 0 045 394**
**EP - A - 0 048 372**
**EP - A - 0 048 373**
**EP - A - 0 088 963**

(73) Patentinhaber: **BASF Aktiengesellschaft, Carl-Bosch-Strasse 38, D-6700 Ludwigshafen (DE)**

(72) Erfinder: **Fischer, Rolf, Dr., Bergstrasse 98, D-6900 Heidelberg (DE)**
Erfinder: **Dockner, Toni, Dr., Grossgasse 6, D-6701 Meckenheim (DE)**
Erfinder: **Rohr, Wolfgang, Dr., In der Dreispitz 13, D-6706 Wachenheim (DE)**
Erfinder: **Stroefer, Eckhard, Dr., Werderstrasse 20, D-6800 Mannheim 1 (DE)**

**EP 0 162 247 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Diese Erfindung betrifft ein Verfahren zur Herstellung von Pyrazolen durch Oxidation von 2-Pyrazolinen mit Wasserstoffperoxid, organischen Peroxiden oder Percarbonsäuren.

Bekanntlich lassen sich Pyrazole durch Oxidation von 2-Pyrazolinen herstellen, wobei man als Oxidationsmittel z.B. Chlor und Hypochlorite (EP-PS 48 373) oder Kaliumpermanganat, Salpetersäure, Luft, Bleitetraacetat oder Chromtrioxid [Weissberger, The Chemistry of Heterocyclic Compounds, Band 22, Seiten 41 bis 49 (1967)] verwendet. Nach den Angaben der EP-PS 45 394 und der EP-A-88 963 stellt man Pyrazole durch Dehydrierung der entsprechenden Pyrazoline mit Schwefel bzw. Selen oder durch katalytische Dehydrierung an einem Katalysator oder Platinmetall in der Gasphase her.

Diese Verfahren haben den Nachteil, dass teure und/oder toxische Oxidationsmittel eingesetzt werden, dass aus den Oxidationsmitteln Produkte entstehen, die zurückgeführt oder entsorgt werden müssen und dass die Ausbeuten an Pyrazolen zum Teil unbefriedigend sind.

Es bestand die Aufgabe, ein Verfahren zur Herstellung von Pyrazolen zu finden, das diese Nachteile nicht aufweist.

Es wurde nun gefunden, dass man Pyrazole der Formel

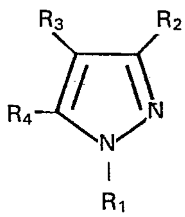 I,

in der die Reste $R_1$ bis $R_4$ Wasserstoffatome, Alkylreste mit 1 bis 6 Kohlenstoffatomen, Phenylreste, die Alkyl- oder Alkoxyreste oder Halogenatome enthalten können, Cyclopentyl-, Cyclohexyl- oder Cycloheptylreste bedeuten, durch Oxidation von 2-Pyrazolinen der Formel

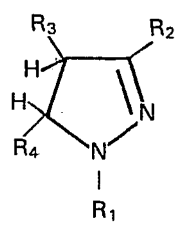 II,

in der $R_1$ bis $R_4$ die obengenannte Bedeutung haben, besonders vorteilhaft dadurch herstellen kann, dass man als Oxidationsmittel eine Verbindung der Formel

$R_5\text{-O-O-H}$ III

verwendet, in der $R_5$ ein Wasserstoffatom, einen Alkylrest mit 4 bis 10 Kohlenstoffatomen oder einen Cyclohexyl-, Cyclohexenyl-, Cumol-, 1-Phenylethyl- oder einen Acylrest $R_6$-CO- bedeutet, wobei $R_6$ für Wasserstoff, einen Alkylrest mit 1 bis 12 Kohlenstoffatomen oder für einen Phenylrest steht.

Die Reaktion lässt sich für den Fall der Herstellung von Pyrazol unter Verwendung von Wasserstoffperoxid durch die folgenden Formeln anschaulich machen:

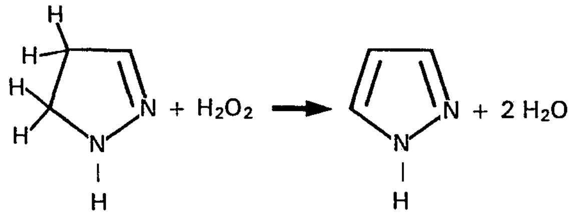


Die als Ausgangsstoffe verwendeten 2-Pyrazoline der Formel II enthalten als Reste $R_1$ bis $R_4$ Wasserstoffatome, Alkylreste mit 1 bis 6 Kohlenstoffatomen, Phenylreste, die gegebenenfalls noch durch Alkyl-, oder Alkoxyreste oder Halogenatome substituiert sein können, Cyclopentyl-, Cyclohexyl- oder Cycloheptylreste. Alkylreste sind z.B. Methyl-, Ethyl-, n-Propyl-, i-Propyl- oder Butylreste.

Beispielsweise seien die folgenden Verbindungen der Formel II genannt:

2-Pyrazolin, 1-Phenyl-2-pyrazolin, 1-Phenyl-5--methyl-2-pyrazolin, 5-Methyl-2-pyrazolin, 4,5-Dimethylpyrazolin, 1,3-Diphenyl-2-pyrazolin, 3-Methyl-2-pyrazolin, 5-Phenyl-2-pyrazolin, 3,4,5-Trimethylpyrazolin, 4-Methyl-2-pyrazolin.

Die Ausgangsverbindungen der Formel II lassen sich z.B. auf bekannte Weise durch Umsetzung von Hydrazin oder 1-Alkyl- oder 1-Arylhydrazinen mit α,β-ungesättigten Aldehyden wie Acrolein oder entsprechend substituierten Acroleinen herstellen.

In den Oxidationsmitteln der Formel III bedeutet $R_5$ z.B. einen Alkylrest mit 4 bis 10 Kohlenstoffatomen, wie einen tert-Butylrest, einen Cyclohexyl-, Cyclohexenyl-, Cumol- oder 1-Phenylethylrest. $R_6$ steht für Wasserstoff, einen Alkylrest mit 1 bis 12 Kohlenstoffatomen, wie für einen Methyl-, Propyl-, Butyl-, Ethyl- oder Dodecylrest. $R_6$ kann auch ein Phenylrest sein.

Im einzelnen seien die folgenden Oxidationsmittel genannt: Wasserstoffperoxid, das bevorzugt als wässrige, 10 bis 90%ige Lösung eingesetzt wird, Percarbonsäuren, wie Perameisensäure, Peressigsäure, Perpropionsäure, Perbenzoesäure oder Per-n--buttersäure, die im allgemeinen als Lösungen in den zugrundeliegenden Carbonsäuren eingesetzt werden und gegebenenfalls noch Wasser enthalten können und Hydroperoxide, wie tert.-Butylhydroperoxid, Ethylbenzolhydroperoxid, Cumolhydroperoxid, Cyclohexylhydroperoxid oder Cyclohexenylhydroperoxid.

Das erfindungsgemässe Verfahren wird zweckmässigerweise in Gegenwart eines unter den Reaktionsbedingungen inerten Lösungsmittels durchgeführt. Als Lösungsmittel sind solche geeignet, in denen sich die 2-Pyrazoline II und wässriges Wasserstoffperoxid, Alkylhydroperoxid bzw. Percarbonsäuren lösen. Solche Lösungsmittel sind z.B. Wasser, Alkohole, wie Methanol, Ethanol, n-Propanol, Isopropanol, 1-Butanol, 2-Butanol, tert.-Butanol,

Amylalkohol, Isoamylalkohol, Cyclohexanol, Ethylenglykol oder Propylenglykol, Carbonsäuren, wie Ameisensäure Essigsäure oder Propionsäure, cyclische Ether, wie Tetrahydrofuran und Dioxan, Oligomere von Ethylenoxid und Propylenoxid oder deren Ether, wie Dimethoxydiethylenglykol, Diethoxyethylenglykol, Diglyme und die Ester, wie die Formiate oder Acetate von gewöhnlichen Alkoholen oder Glykolen. Pro Mol Ausgangsverbindung II verwendet man zweckmässigerweise 0,1 bis 80 Mol, insbesondere 2 bis 60 Mol des Lösungsmittels.

Das neue Verfahren wird z.B. so durchgeführt, dass man pro Mol 2-Pyrazolin II 0,3 bis 2 Mole, insbesondere 0,5 bis 1,3 Mole des Oxidationsmittels III einsetzt. Man arbeitet zweckmässig bei Temperaturen zwischen 20 und 150°C, insbesondere bei 50 bis 100°C. Das Verfahren kann chargenweise oder kontinuierlich drucklos oder unter Druck durchgeführt werden. Unumgesetzte Pyrazoline II können gegebenenfalls nach der Umsetzung destillativ von den entstandenen Pyrazolen I abgetrennt und erneut für die erfindungsgemässe Umsetzung verwendet werden.

Die Umsetzung kann man bei diskontinuierlicher Arbeitsweise z.B. wie folgt vornehmen: Zu einer Lösung des Pyrazolins II in dem jeweiligen Lösungsmittel gibt man bei der Reaktionstemperatur die notwendige Menge der Perverbindung III. Nach beendeter Zugabe wird so lange bei der Reaktionstemperatur nachgerührt, bis die Perverbindung praktisch vollständig umgesetzt ist. Nach dem Abkühlen des Reaktionsgemisches werden Wasser, Lösungsmittel, gebildetes Pyrazol I und gegebenenfalls unumgesetztes Pyrazolin II und Carbonsäure durch fraktionierte Destillation voneinander getrennt. Es ist auch möglich, das Pyrazol I durch Extraktion aus dem Reaktionsgemisch abzutrennen.

Die nach dem erfindungsgemässen Verfahren zugänglichen Pyrazole I stellen wertvolle Zwischenprodukte, z.B. für die Herstellung von Farbstoffen, Pharmazeutika und Pflanzenschutzmitteln dar.

Gegenüber den bekannten Verfahren zur Herstellung von Pyrazolen I zeichnet sich das erfindungsgemässe Verfahren durch den grossen Vorteil aus, dass man anstelle von teuren und/oder toxischen Oxidationsmitteln Wasserstoffperoxid, Hydroperoxide oder Percarbonsäuren verwenden kann. Da hierbei aus den Oxidationsmitteln lediglich Wasser, Alkohole oder Carbonsäuren entstehen, treten weder Rückführungs- noch Entsorgungsprobleme auf. Das vorteilhafte Ergebnis des Verfahrens der Erfindung ist überraschend, da nicht vorherzusehen war, dass unter den Reaktionsbedingungen überwiegend Pyrazole I entstehen. Es ist nämlich bekannt, dass cyclische sekundäre Amine mit Wasserstoffperoxid zu den entsprechenden Aminoxiden oder Hydroxylaminen oxidiert werden können [Zh. Obshch. Khim. <u>30</u>, Seite 1631 (1960)].

*Beispiel 1*

Zu einer Lösung von 70 g 2-Pyrazolin (1 Mol) in 700 ml Wasser wurden bei 100°C innerhalb von 15 Minuten 68 g Wasserstoffperoxid, 50%ig (1 Mol) gegeben. Das Gemisch wurde 25 Minuten bei 100°C nachgerührt. Durch iodometrische Titration wurde festgestellt, dass der Peroxidgehalt nach dieser Zeit auf 3% der insgesamt zugesetzten $H_2O_2$-Menge abgesunken war. Die gaschromatographische Analyse zeigte, dass im Reaktionsgemisch 41 g Pyrazol (0,6 Mol) und 5,4 g unumgesetztes 2-Pyrazolin (0,08 Mol) enthalten waren. Die Ausbeute an Pyrazol beträgt demnach 60%, die Selektivität 65%.

Durch fraktionierte Destillation des Reaktionsgemisches wurden 38 g Pyrazol (56% Ausbeute) von Siedepunkt 85 bis 90°C/20 mbar erhalten.

*Beispiel 2*

Zu einer Lösung von 3,5 g 2-Pyrazolin (50 mmol) in 35 mm Wasser wurden bei 75 ± 2°C innerhalb von zwei Minuten 1,7 g 50%iges Wasserstoffperoxid (25 mmol) gegeben. Das Gemisch wurde eine Stunde bei gleicher Temperatur nachgerührt. Der Peroxidgehalt betrug nach dieser Zeit 4%, bezogen auf insgesamt zugesetztes Wasserstoffperoxid. Die gaschromatographische Analyse zeigte, dass im Reaktionsgemisch 1,05 g Pyrazol (15,5 mmol) und 1,8 g unumgesetztes 2-Pyrazolin (26 mmol) enthalten waren. Die Ausbeute an Pyrazol beträgt demnach 31%, die Selektivität 65%.

*Beispiel 3*

Zu einer Lösung von 3,5 g 2-Pyrazolin (50 mmol) in 35 ml Dioxan wurden bei 90 ± 2°C innerhalb von drei Minuten 5,1 g 50%iges Wasserstoffperoxid (75 mmol) gegeben. Das Gemisch wurde 5 Stunden bei gleicher Temperatur nachgerührt. Der Peroxidgehalt betrug nach dieser Zeit 44%, bezogen auf insgesamt zugesetztes Wasserstoffperoxid. Die gaschromatographische Analyse zeigte, dass das 2-Pyrazolin vollständig umgesetzt war und 2 g Pyrazol (29 mmol) entstanden waren (Pyrazol-Ausbeute 59%).

*Beispiel 4*

Ein Gemisch aus 50 g Eisessig, 5,2 g 50%igem Wasserstoffperoxid und 0,25 g konzentrierter Schwefelsäure wurde 48 Stunden lang bei Raumtemperatur gerührt. 26 g der so hergestellten «Gleichgewichtsperessigsäure» wurden zur Neutralisation der Schwefelsäure vor ihrer Verwendung mit 0,28 g Kaliumacetat versetzt.

Zu einer Lösung von 2,1 g 5-Methyl-2-pyrazolin (25 mmol) in 17 ml Wasser wurden bei 100°C innerhalb von 4 Minuten 19,1 g der vorher hergestellten Peressigsäure (9,94 Gew.-%) (25 mmol) getropft. Das Reaktionsgemisch wurde 5 Minuten bei 100°C gerührt. Nach dieser Zeit war durch iodometrische Titration keine Peressigsäure mehr nachweisbar. Nach Neutralisation mit wässriger Natriumcarbonatlösung zeigte die gaschromatographische Analyse, dass im Reaktionsgemisch 0,82 g 5-Methylpyrazol (10 mmol) und 0,29 g unumgesetztes 5-Methyl-2--pyrazolin (3,5 mmol) enthalten waren. Die Ausbeute an 5-Methylpyrazol betrug demnach 40%, die Selektivität 47%.

*Beispiel 5*

4,4 g (30 mmol) 1-Phenyl-2-pyrazolin wurden in 42 g eines n-Propanol/Wasser-Gemisches (Volumenverhältnis 1 : 1) gelöst. Zu dieser Lösung wurden bei 90°C innerhalb von 2 Minuten 2,1 l 50%iges Wasserstoffperoxid (31 mmol) gegeben. Das Reaktionsgemisch wurde 3,5 Stunden bei 90 ± 2°C gerührt. Nach dieser Zeit waren 94% des eingesetzten Wasserstoffperoxids umgesetzt. Die gaschromatographische Analyse ergab, dass im Reaktionsgemisch 2,7 g 1-Phenylpyrazol (19 mmol) und 0,7 g unumgesetztes 1-Phenyl-2-pyrazolin (5 mmol) enthalten waren. Die Ausbeute an 1-Phenylpyrazol betrug demnach 63%, die Selektivität 75%.

*Beispiel 6*

Beispiel 5 wurde mit einer Lösung von 4,4 g (30 mmol) 1-Phenyl-2-pyrazolin in 40 g Dioxan wiederholt, wobei 3,4 g tert.-Butylhydroperoxid (80%ig) (30 mmol) bei 100°C innerhalb von 2 Minuten zugetropft wurden. Das Reaktionsgemisch wurde 6,5 Stunden bei 100 ± 2°C gerührt. Gaschromatographisch wurden nach dieser Zeit 3 g 1-Phenylpyrazol (21 mmol) und 1 g unumgesetztes 1-Phenyl-2-pyrazolin (2 mmol) gefunden. Die Ausbeute an 1-Phenylpyrazol betrug demnach 70%, die Selektivität 91%.

*Beispiel 7*

Zu einer Lösung von 4,2 g 3-Methyl-2-pyrazolin (50 mmol) in 35 ml Dioxan wurden bei 100°C innerhalb von 5 Minuten 27,3 wasserfreie Peressigsäure (hergestellt nach Houben-Weyl, Methoden der organischen Chemie, 4. Auflage, Band 8, Seite 41) (13,9 Gew.-%) (50 mmol) getropft. Das Reaktionsgemisch wurde weitere 5 Minuten bei dieser Temperatur gerührt. Nach dieser Zeit war durch iodometrische Titration keine Peressigsäure mehr nachweisbar. Nach Neutralisation mit wässriger Natriumcarbonatlösung zeigte die gaschromatographische Analyse, dass im Reaktionsgemisch 2,5 g 3-Methylpyrazol (30 mmol) und 0,4 g unumgesetztes 3-Methyl-2-pyrazolin (5 mmol) enthalten waren. Die Ausbeute an 3-Methylpyrazol beträgt 60%, die Selektivität 67%.

*Beispiel 8*

Beispiel 7 wurde unter gleichen Bedingungen mit gleichen Mengen 4-Methyl-2-pyrazolin anstelle von 3-Methyl-2-pyrazolin wiederholt. Die gaschromatographisch ermittelte Ausbeute an 4-Methylpyrazol betrug 57%, die Selektivität 61%.

## Patentansprüche

1. Verfahren zur Herstellung von Pyrazolen der Formel

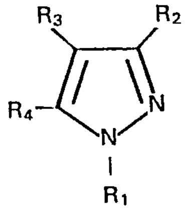

I,

in der die Reste $R_1$ bis $R_4$ Wasserstoffatome, Alkylreste mit 1 bis 6 Kohlenstoffatomen, Phenylreste, die Alkyl- oder Alkoxyreste oder Halogenatome enthalten können, Cyclopentyl-, Cyclohexyl- oder Cycloheptylreste bedeuten, durch Oxidation von 2-Pyrazolinen der Formel

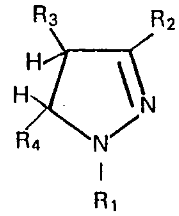

II,

in der $R_1$ bis $R_4$ die obengenannte Bedeutung haben, dadurch gekennzeichnet, dass man als Oxidationsmittel eine Verbindung der Formel

$R_5$-O-O-H III

verwendet, in der $R_5$ ein Wasserstoffatom, einen Alkylrest mit 4 bis 10 Kohlenstoffatomen oder einen Cyclohexyl-, Cyclohexenyl-, Cumol-, 1-Phenylethyl- oder einen Acylrest $R_6$-CO- bedeutet, wobei $R_6$ für Wasserstoff, einen Alkylrest mit 1 bis 12 Kohlenstoffatomen oder für einen Phenylrest steht.

2. Verfahren nach Anspruch 1, dadurch gekennzeichnet, dass man als Oxidationsmittel eine wässrige 10 bis 90gew.%ige Wasserstoffperoxidlösung verwendet.

3. Verfahren nach Anspruch 1, dadurch gekennzeichnet, dass man die Oxidation bei Temperaturen zwischen 20 und 150°C vornimmt.

4. Verfahren nach Anspruch 1, dadurch gekennzeichnet, dass man pro Mol des Pyrazolins der Formel II 0,3 bis 2 Mol des Oxidationsmittels anwendet.

## Claims

1. A process for the preparation of a pyrazole of the formula

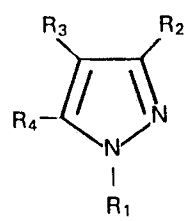

I,

where $R_1$, $R_2$, $R_3$ and $R_4$ are each hydrogen; alkyl of 1 to 6 carbon atoms; phenyl optionally containing alkyl or alkoxy radicals or halogen atoms; cyclopentyl; cyclohexyl; or cycloheptyl, by oxidation of a 2-pyrazoline of the formula

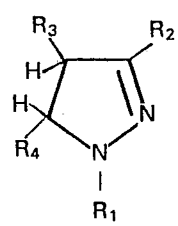

II,

where $R_1$, $R_2$, $R_3$ and $R_4$ have the above meanings, wherein there is used as oxidizing agent a compound of the formula

$R_5$-O-O-H III,

where $R_5$ is hydrogen, alkyl of 4 to 10 carbon atoms, cyclohexyl, cyclohexenyl, cumyl, 1-phenylethyl or an acyl radical $R_6$-CO- in which $R_6$ is hydrogen, alkyl of 1 to 12 carbon atoms or phenyl.

2. A process as claimed in Claim 1, wherein an aqueous 10-90% strength by weight hydrogen peroxide solution is used as the oxidizing agent.

3. A process as claimed in Claim 1, wherein the oxidation is carried out at a temperature of from 20 to 150°C.

4. A process as claimed in Claim 1, wherein from 0.3 to 2 moles of the oxidizing agent are used per mole of the pyrazoline of the formula II.

**Revendications**

1. Procédé de préparation de pyrazoles de la formule

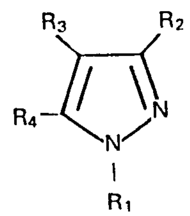

I,

dans laquelle les substituants $R_1$ à $R_4$ désignent des atomes d'hydrogène, des radicaux alkyle en $C_1$ à $C_6$, des groupes phényle pouvant porter des radicaux alkyle ou alcoxy ou des atomes d'halogènes, des groupes cyclopentyle, cyclohexyle ou cycloheptyle, par oxydation de 2-pyrazolines de la formule

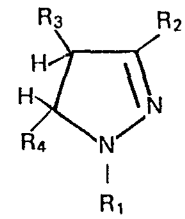

II,

dans laquelle $R_1$ à $R_4$ possèdent la signification définie, caractérisé en ce que l'on emploie comme agent d'oxydation un composé de la formule

$R_5$-O-O-H III,

dans laquelle $R_5$ désigne un atome d'hydrogène, un radical alkyle en $C_4$ à $C_{10}$, un groupe cyclohexyle, cyclohexényle, cumyle, 2-phényl-éthyl ou un groupe acyle $R_6$-CO-, où $R_6$ désigne un atome d'hydrogène, un radical alkyle en $C_1$ à $C_{12}$ ou un groupe phényle.

2. Procédé suivant la revendication 1, caractérisé en ce que l'on emploie comme agent d'oxydation une solution aqueuse de peroxyde d'hydrogène d'une concentration de 10 à 90% en poids.

3. Procédé suivant la revendication 1, caractérisé en ce que l'oxydation est réalisée à des températures comprises entre 20 et 150°C.

4. Procédé suivant la revendication 1, caractérisé en ce que l'on met en oeuvre entre 0,3 à 2 moles d'agent d'oxydation par mole de la pyrazoline de la formule II.